# EUROPÄISCHE PATENTSCHRIFT

(11) **EP 0 949 882 B1**
(45) Veröffentlichungstag und Bekanntmachung des Hinweises auf die Patenterteilung: **02.07.2003**
(21) Anmeldenummer: 98954470.5
(22) Anmeldetag: 31.10.1998
(51) Int. Cl.: A61B 17/32

(54) **ENDOSKOPISCHES INSTRUMENT MIT SCHNEIDWERKZEUG**
ENDOSCOPIC INSTRUMENT WITH A CUTTING TOOL
INSTRUMENT ENDOSCOPIQUE A ORGANE COUPANT

(30) Priorität: 04.11.1997 DE 19748579
(43) Veröffentlichungstag der Anmeldung: 20.10.1999
(73) Patentinhaber: Karl Storz GmbH & Co. KG, 78532 Tuttlingen (DE)
(72) Erfinder: DITTRICH, Horst, D-78194 Immendingen (DE); WELLER, Thomas, D-78532 Tuttlingen (DE); DUDDA, Werner, D-04860 Süptitz (DE)
(74) Vertreter: Heuckeroth, Volker, Dr.
(86) Internationale Anmeldenummer: EP9806898
(87) Internationale Veröffentlichungsnummer: WO99022654

(56) Entgegenhaltungen:
- EP-A- 0 638 279
- DE-A- 2 737 014
- DE-A- 3 707 403
- DE-A- 3 741 938
- US-A- 5 258 026

## Beschreibung

Die Erfindung betrifft ein endoskopisches Instrument, mit einem Schaft zum Einführen in kanalartige Körpergänge, wie Rektum, Trachea, Ösophagus, sowie mit einer Endoskopoptik.

Ein derartiges Instrument ist aus dem Firmenprospekt der Firma Karl Storz GmbH & Co., Tuttlingen: "STORZ, THE WORLD OF ENDOSCOPY, PROCTOLOGY, FIFTH EDITION 1/92", 1992, Seite PRO 2, bekannt. Das dort beschriebene Instrument ist ein Proktoskop.

Ein Proktoskop wird in der Medizin zur Inspektion des Analkanals und des unteren Abschnitts des Rektums eines Patienten verwendet. Dazu wird das Proktoskop mit einem in einem Tubus eingeschobenen Obturator, dessen distales Ende abgeschrägt ausgebildet ist, in den Analkanal eingeführt. Der Obturator wird danach wieder aus dem Tubus herausgezogen und stattdessen wird ein seitlich gefensterter Einsatz in den Tubus eingeführt, mit dem der Analkanal über eine Endoskopoptik inspiziert werden kann, um proktologische Erkrankungen festzustellen.

Wird bei der proktologischen Untersuchung ein Tumor in der Darmwand festgestellt, so muß dieser anschließend operativ entfernt werden. Zur operativen Entfernung von tiefen Tumoren im vorderen Rektum ist es notwendig, einen kreisförmigen Schnitt in Umfangsrichtung in die Darmwand einzubringen. Diese Operation ist sehr schwierig und zeitaufwendig und kann mit dem herkömmlichen Proktoskop allein nicht durchgeführt werden, sondern erfordert ein spezielles chirurgisches Schneidinstrument.

Die Erfindung ist jedoch nicht auf ein solches Proktoskop beschränkt. Weitere vergleichbare endoskopische Instrumente, die in kanalartige Körpergänge eingeführt werden, um den Körpergang zu inspizieren, sind das Rektoskop, Ösophagoskop und Tracheoskop. Rektoskope werden ebenfalls zur Inspektion des Darms in den Darm eingeführt. Mit einem Ösophagoskop wird die Speiseröhre inspiziert, mit einem Tracheoskop die Luftröhre. Auch unter diesen endoskopischen Instrumenten sind derzeit nur solche erhältlich, mit denen die entsprechenden Körpergänge inspiziert werden können, mit denen es jedoch nicht möglich ist, einen kreisförmigen Schnitt in die Wand des jeweiligen Körperganges einzubringen.

Aus der DE-A-27 37 014 ist ein Endoskop zum Aufschlitzen von Harnröhren-Strikturen bekannt. Das Endoskop weist ein Schaftrohr auf, in dem distal ein Messer zum Aufschlitzen der Striktur vorgesehen ist. Das Messer ist aus einer Ruhelage, in der das Messer im Schaft versenkt liegt, durch einen distalseits von ihm angeordneten, längsverschieblichen Keil mittels einer proximalen Handhabe über einen Zugdraht um eine Querachse durch einen Längsschlitz des Schafts heraus verschwenkbar. Mit dem Messer wird ein Schnitt in Längsrichtung der Schaftachse ausgeführt.

Ferner ist aus der US-A-4 674 500 ein chirurgisches Schneidinstrument bekannt, das in der Arthroskopie und bei solchen Operationsverfahren verwendet wird, wo der Zugang zu dem Operationsort begrenzt oder schwierig ist. Dieses Instrument weist dementsprechend einen sehr dünnen und am distalen Ende seitlich abgebogenen Schaft auf, in dem eine Messerklinge in Längsrichtung des Schaftes verschiebbar aufgenommen ist. Das Messer ist biegsam ausgebildet, so daß es durch Vorschieben aus dem seitlich abgekrümmten distalen Ende des dünnen Schaftes herausgeschoben werden kann, um einen Schnitt auszuführen. Dieses Instrument mit einem abgekrümmten Schaft eignet sich jedoch nicht zur Verwendung in einem kanalartigen Körpergang.

Der Erfindung liegt daher die Aufgabe zugrunde, ein endoskopisches Instrument der eingangs genannten Art dahingehend zu verbessern, daß mit dem Instrument nicht nur eine Inspektion des jeweiligen Körperganges, sondern auch eine operative Entfernung bspw. eines Tumors durch Einbringen eines Schnittes in die Wand des Körperganges möglich ist.

Hinsichtlich des eingangs genannten Instruments wird diese Aufgabe erfindungsgemäß dadurch gelöst, daß in dem Schaft ein Schneidwerkzeug zum Einbringen eines Schnittes in umfänglicher Richtung des Körperganges angeordnet ist, das über einen Verstellmechanismus aus dem Schaft über den Umfang des Schafts hinaus ausfahrbar und wieder in diesen einfahrbar ist.

Dadurch, daß in das erfindungsgemäße Instrument in dem Schaft ein Schneidwerkzeug integriert ist, kann es nicht nur zu Inspektionszwecken, sondern auch als Operationsinstrument zum Herausschneiden bspw. von Tumoren eingesetzt werden. Die Operation kann dabei vorteilhaft mit der Endoskopoptik beobachtet und kontrolliert werden. Dadurch, daß das Schneidwerkzeug über den Umfang des Schafts hinaus ausfahrbar ist, kann der erforderliche Schnitt, bspw. im Falle einer Ausbildung des erfindungsgemäßen Instruments als Proktoskop in Umfangsrichtung der Darmwand ausgeführt werden, indem der Schaft bei ausgefahrenem Schneidwerkzeug um seine Längsachse gedreht wird, wodurch das Schneidwerkzeug einen Schnitt einer Tiefe entsprechend der Ausfahrweite bzw. entsprechend der Weite, in der das Schneidwerkzeug über den Umfang des Schafts hinausragt, in die Darmwand einbringt. Dadurch, daß das Schneidwerkzeug in den Schaft einfahrbar und damit versenkbar ist, kann der Schaft problemlos in den Körpergang eingeführt werden, ohne daß die Wand des Gangs durch das Schneidwerkzeug beim Einführen des Instruments verletzt wird. Als Schneidwerkzeuge können Klingen zum mechanischen Schneiden, Drahtelektroden zum Schneiden mit HF-Strom, Ultraschall-Schneideapplikatoren oder Laser verwendet werden. Unter "ausfahrbar" wird im Sinne des Gegenstands der vorliegenden Erfindung bspw. eine radiale Bewegung oder eine Ausschwenk- oder Biege-Bewegung des Schneidwerkzeuges verstanden, oder auch das Einschalten, Freigeben oder Erweitern des Wirkradius eines Laser-Schneidwerkzeugs.

Somit wird die der Erfindung zugrundeliegende Aufgabe vollkommen gelöst.

In einer bevorzugten Ausgestaltung ist das Schneidwerkzeug in der maximal ausgefahrenen und in der eingefahrenen Position arretierbar.

Durch die Arretierbarkeit des Schneidwerkzeuges in der maximal ausgefahrenen Position kann der auszuführende Schnitt sicher durchgeführt werden, ohne daß die Gefahr besteht, daß das Schneidwerkzeug beim Schneiden in den Schaft zurückgeschoben wird. Somit kann der Schnitt betriebssicher durchgeführt werden. Durch die Arretierbarkeit der eingefahrenen Position wird gewährleistet, daß das Schneidwerkzeug beim Einführen des Schafts in den Körpergang nicht vorzeitig ausfährt und im Falle einer Verwendung des Instruments zu Inspektionszwecken nicht unerwünscht ausfährt und bspw. die Darmwand verletzt. Somit ist die Betriebssicherheit des erfindungsgemäßen Instruments verbessert.

In einer weiteren bevorzugten Ausgestaltung ist das Schneidwerkzeug in einer Vielzahl von Positionen unterschiedlicher Ausfahrweite zwischen der zurückgefahrenen und der maximal ausgefahrenen Position arretierbar.

Diese Maßnahme hat den Vorteil, daß mit dem erfindungsgemäßen Instrument je nach den Erfordernissen bei der Entfernung bspw. eines Tumors Schnitte unterschiedlicher Tiefe in die Wand des Körperganges eingebracht werden können, wobei sichergestellt ist, daß das Schneidwerkzeug in seiner eingestellten Ausfahrweite arretiert ist, bis eine neue Ausfahrweite eingestellt wird, um eine andere Schnittiefe zu erzielen.

Bei einem bevorzugten Ausführungsbeispiel ist das Schneidwerkzeug radial ausfahrbar.

Ein radial ausfahrbares Schneidwerkzeug hat den Vorteil, daß das Schneidwerkzeug an einem exakt definierten Punkt des Umfanges der Wand des Körperganges, wo der Schnitt beginnen soll, angesetzt werden kann. Dazu wird der Schaft in dem Körpergang genau mit der Umfangsstelle, an der das Schneidwerkzeug aus dem Schaft austritt, an diesen Punkt bewegt, wobei das Schneidwerkzeug dann beim Ausfahren genau an dem Punkt in die Wand des Körperganges eingreift. Somit ist die Präzision des erfindungsgemäßen Instruments erhöht.

In einer weiteren bevorzugten Ausgestaltung ist der Verstellmechanismus vom proximalen Ende des Instruments aus betätigbar.

Diese Maßnahme hat den Vorteil, daß der operierende Arzt das Aus- und Einfahren des Schneidwerkzeugs leicht betätigen kann, wodurch die Handhabung des erfindungsgemäßen Instruments verbessert ist.

In einer weiteren bevorzugten Ausgestaltung ist der Verstellmechanismus zum Arretieren des Schneidwerkzeuges verrastbar.

Der Verstellmechanismus sorgt somit nicht nur für das Aus- und Einfahren des Schneidwerkzeuges, sondern gleichzeitig für die Arretierung des Schneidwerkzeuges. Dadurch wird der Vorteil erzielt, daß zur Arretierung des Schneidwerkzeuges keine weiteren Mittel vorgesehen werden müssen. Eine Verrastung hat darüber hinaus den Vorteil, einerseits schnell lösbar zu sein, und andererseits kann das Schneidwerkzeug rasch und sicher arretiert werden.

Bei einem bevorzugten Ausführungsbeispiel weist der Verstellmechanismus einen proximal betätigbaren Betätigungsstab auf, dessen distales Ende mit dem Schneidwerkzeug zusammenwirkt.

Diese Maßnahme hat den Vorteil, daß der das Instrument bedienende Arzt das Schneidwerkzeug am proximalen Ende des erfindungsgemäßen Instrumentes betätigen kann, wodurch die Handhabung des erfindungsgemäßen Instrumentes verbessert wird.

Dabei ist es bevorzugt, wenn der Betätigungsstab drehbar ist und das Schneidwerkzeug durch Drehen des Betätigungsstabes aus- und einfahrbar ist.

Ein Verstellmechanismus mit einem drehbaren Betätigungsstab hat den Vorteil, daß ein solcher Verstellmechanismus einfach zu bedienen ist.

Weiterhin ist es bevorzugt, wenn das proximale Ende des Betätigungsstabes zwei relativ zueinander verdrehbare Räder aufweist, wobei das eine Rad drehfest mit dem Betätigungsstab verbunden ist und das andere Rad drehfest an dem Schaft befestigbar ist.

Diese Maßnahme hat den Vorteil, daß der Betätigungsstab mit dem drehfest an dem Schaft befestigbaren Rad an dem Schaft fixiert werden kann, während das drehfest mit dem Betätigungsstab verbundene Rad dazu verwendet werden kann, durch Drehen das Schneidwerkzeug aus- und einzufahren. Das drehfest an dem Schaft befestigbare Rad braucht zum Herausnehmen des Betätigungsstabes aus dem Schaft nur gelöst zu werden und ermöglicht somit eine einfache Zerlegung des erfindungsgemäßen Instruments.

Weiterhin ist es dabei bevorzugt, wenn zwischen den Rädern eine oder mehrere Kugelrasten angeordnet sind.

Mit den Kugelrasten wird auf vorteilhafte Weise ein mechanisch einfacher Verrastmechanismus zum Arretieren des Schneidwerkzeuges geschaffen.

Alternativ dazu ist es bevorzugt, wenn der Betätigungsstab axial verschiebbar ist und das Schneidwerkzeug durch Verschieben des Betätigungsstabes ein- und ausfahrbar ist.

Diese Maßnahme führt ebenfalls zu einem leicht bedienbaren Verstellmechanismus.

In einem bevorzugten Ausführungsbeispiel ist der Betätigungsstab mit dem Schneidwerkzeug durch eine axial lösbare Steckverbindung verbunden.

Diese Maßnahme hat den Vorteil, daß sich Betätigungsstab und Schneidwerkzeug einfach durch Einschieben des Betätigungsstabes in den Schaft miteinander verbinden lassen, und umgekehrt braucht der Betätigungsstab zum Auswechseln des Schneidwerkzeuges nur zurückgezogen zu werden, wodurch das Schneidwerkzeug freigegeben wird.

Bei einer weiteren bevorzugten Ausgestaltung ist ein Objektiv der Endoskopoptik im Bereich des Schneidwerkzeuges derart angeordnet, daß das Schneidwerkzeug durch die Endoskopoptik beobachtbar ist.

Durch diese konstruktive Maßnahme kann der operierende Arzt sowohl das Aus- bzw. und Einfahren des Schneidwerkzeuges als auch den Schneidvorgang selbst beobachten und kontrollieren.

In einer weiteren bevorzugten Ausgestaltung ist das Schneidwerkzeug in der ausgefahrenen Position über einen Antrieb in Oszillation versetzbar.

Die oszillierende Hin- und Herbewegung des Schneidwerkzeuges bewirkt auf vorteilhafte Weise eine Erhöhung der Schneidwirkung des Schneidwerkzeuges.

Bei einer weiteren bevorzugten Ausgestaltung ist eine Anzeigevorrichtung für die Ausfahrweite des Schneidwerkzeuges vorgesehen.

Diese Maßnahme hat den Vorteil, daß der das Instrument bedienende Arzt die Ausfahrweite des Schneidwerkzeuges kontrollieren und exakt auf die jeweilige Schnittiefe einstellen kann. Dies ist insbesondere deswegen von Vorteil, weil der Arzt zwar durch die Endoskopoptik das Schneidwerkzeug beobachten kann, das Schneidwerkzeug je nach verwendeter Endoskopoptik, bzw. je nach deren Lage zum Schneidwerkzeug, jedoch vergrößert oder verkleinert abgebildet wird, wodurch der Arzt irrtümlicherweise eine unzutreffende Ausfahrweite einstellen könnte. Die Anzeigevorrichtung beugt einem derartigen Irrtum vor.

Dabei ist es bevorzugt, wenn die Anzeigevorrichtung am Schneidwerkzeug vorgesehen ist.

Dies kann bspw. durch eine Skala an dem Schneidwerkzeug erreicht werden, die dann durch die Endoskopoptik abgelesen werden kann.

In einer weiteren bevorzugten Ausgestaltung ist die Anzeigevorrichtung am Verstellmechanismus vorgesehen.

Auch mit dieser Maßnahme wird eine exakte Kontrolle der Ausfahrweite des Schneidwerkzeuges ermöglicht. Es ist auch durchaus möglich, eine Anzeigevorrichtung sowohl am Schneidwerkzeug als auch am Verstellmechanismus vorzusehen.

Bei einem bevorzugten Ausführungsbeispiel ist das Schneidwerkzeug eine Klinge, deren Schneidkante in Umfangsrichtung des Schafts gerichtet ist.

Die Verwendung einer Klinge als Schneidwerkzeug stellt vorteilhafterweise eine mechanisch einfache stabile und kostengünstige Ausführung des erfindungsgemäßen Instruments dar, mit der der gewünschte kreisförmige Schnitt in Umfangsrichtung der Wand des Körperganges durchgeführt werden kann.

In einer bevorzugten Ausgestaltung verjüngt sich die Klinge im Bereich der Schneidkante zu einer Spitze.

Diese Maßnahme hat den Vorteil, daß die Klinge beim Ausfahren aus dem Schaft mit ihrer Spitze leicht in die Wand des Körperganges eindringen kann.

In einer weiteren bevorzugten Ausgestaltung weist die Klinge ein Langloch auf, in das ein am distalen Ende des Betätigungsstabes exzentrisch zu dessen Längsachse angeordneter Stift eingreift.

Durch diese Maßnahme wird auf vorteilhafte Weise eine besonders einfache Art einer Steckverbindung zwischen dem Betätigungsstab und der Klinge geschaffen. Durch die exzentrische Anordnung des Stiftes wird eine Drehbewegung des Betätigungsstabes in eine radiale Bewegung der Klinge umgesetzt. Auf diese Weise wird ein besonders einfacher Verstellmechanismus zum Ein- und Ausfahren der Klinge für das erfindungsgemäße Instrument geschaffen.

Weiterhin ist es bevorzugt, wenn die Klinge in einer zum Umfang des Schafts hin offenen, im wesentlichen radial verlaufenden Ausnehmung des Schafts aufgenommen ist.

Im eingefahrenen Zustand nimmt die Ausnehmung die Klinge vollständig auf. Die Ausnehmung stellt darüber hinaus eine mechanisch einfache radiale Führung beim Ein- und Ausfahren der Klinge dar. Ein weiterer Vorteil besteht darin, daß die Klinge beim Schneiden in der Ausnehmung in Schneidrichtung sicher gehalten wird.

In einer weiteren bevorzugten Ausgestaltung ist der Schaft als Einsatz ausgebildet, der in einen Tubus einschiebbar ist.

Dies hat den Vorteil, daß zuerst der Tubus in den Körpergang, sodann der Einsatz zum Ausführen des Schnitts in den Tubus und danach ggf. andere Instrumente in den Tubus eingeführt werden können, bspw. zum Entfernen des herausgeschnittenen Gewebes.

Dabei ist es bevorzugt, wenn der Einsatz in dem Tubus drehbar befestigbar ist.

Diese Maßnahme hat bspw. bei einer Ausbildung des Instruments als Proktoskop den Vorteil, daß der Einsatz axial an dem Tubus gesichert werden kann, gleichzeitig kann der Einsatz jedoch zum Inspizieren der Darmwand und zum Durchführen des Schnittes in der Darmwand in dem Tubus gedreht werden.

In einer weiteren bevorzugten Ausgestaltung ist das Schneidwerkzeug ein Schneidedraht.

Bei dieser Ausgestaltung des erfindungsgemäßen Instrumentes wird der Schnitt durch HF-Strom bewirkt.

In einer weiteren bevorzugten Ausgestaltung ist der Schaft flexibel.

Diese Ausgestaltung hat den Vorteil, daß sich der Schaft an den anatomischen Verlauf des Körperganges, in den er eingeführt wird, anpassen kann.

Weitere Vorteile ergeben sich aus der nachfolgenden Beschreibung der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung wird hiernach mit Bezug auf die Zeichnung näher beschrieben. Es zeigen:
- Fig. 1: eine perspektivische Ansicht eines als Proktoskop ausgebildeten endoskopischen Instruments mit ausgefahrenem Schneidwerkzeug, vom distalen Ende aus gesehen;
- Fig. 2: den distalen Bereich des Proktoskops in Fig. 1 mit eingefahrenem Schneidwerkzeug;
- Fig. 3: eine perspektivische Ansicht des Proktoskops in Fig. 1, vom proximalen Ende aus gesehen;
- Fig. 4: den distalen Bereich des Proktoskops in Fig. 1 bis 3 mit einer distalen Abdeckung;
- Fig. 5: eine auseinandergezogene Darstellung des Proktoskops in Fig. 1 bis 4;
- Fig. 6: eine perspektivische Detailansicht eines Betätigungsstabes sowie ein Schneidwerkzeug des Proktoskops in Fig. 1 bis 5 ; und
- Fig. 7a) und 7b): das Funktionsprinzip des Verstellmechanismus zum Ein- und Ausfahren des Schneidwerkzeuges.

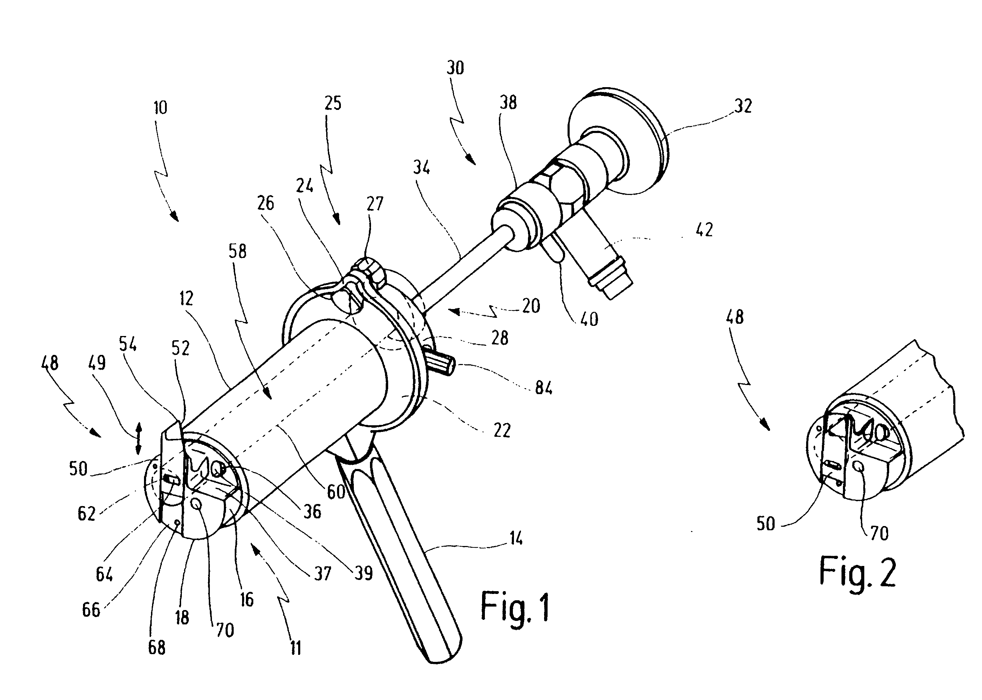

In Fig. 1 bis 5 ist ein endoskopisches Instrument dargestellt, das als mit dem allgemeinen Bezugszeichen 10 versehenes Proktoskop ausgebildet ist.

Das Proktoskop 10 wird zur Inspektion des Analkanals und des Rektums sowie zur operativen Entfernung von Tumoren im Rektum verwendet.

Das Proktoskop 10 weist einen Schaft 11 sowie einen Tubus 12 in Form eines im wesentlichen zylindrischen Rohres auf. An dem Tubus 12 ist ein Griff 14 in Form eines Sechskant-Stabes befestigt. Der Griff 14 kann in eine hier nicht dargestellte Halterung eingesetzt und in dieser fixiert werden.

Der Schaft 11 ist als Einsatz 16 ausgebildet, der in den Tubus 12 eingeschoben ist. Bei der in Fig. 1 bis 4 vollständig eingeschobenen Position des Einsatzes 16 ragt ein distales Ende 18 des Einsatzes 16 axial aus dem Tubus 12 vor. Ein proximales Ende 20 des Einsatzes 16 ist an einem proximalen Ende 22 des Tubus 12 drehbar befestigt. Das proximale Ende 22 des Tubus 12 ist dazu als sich nach proximal erweiternder Konus ausgebildet, über dessen Rand ein Ringflansch 24 des Einsatzes 16 geschoben ist. In dem Ringflansch 24 ist ein Befestigungsmittel 25 angeordnet, das ein Klemmstück 26 und einen damit verbundenen Drehknopf 27 aufweist. Das Klemmstück 26 wird zur Befestigung des Einsatzes 16 an dem Tubus 12 über das konische proximale Ende 22 des Tubus 12 gedreht. Dadurch ist der Einsatz 16 in den Tubus 12 axial fixiert, kann jedoch über einen Vollkreis in dem Tubus 12 gedreht werden.

Zum Drehen des Einsatzes 16 in dem Tubus 12 ist an dem Einsatz 16 ein fest damit verbundenes großes gerändeltes Rad 28 angeordnet.

Das Proktoskop 10 weist weiterhin eine Endoskopoptik 30 auf. Die Endoskopoptik 30 weist ein Okular 32 am proximalen Ende des Proktoskops auf. Die Endoskopoptik 30 ist in einen Kanal 34 eingeschoben und aus diesem herausnehmbar. Der Kanal 34 ist fest mit dem Einsatz 16 verbunden. Der Kanal 34 geht durch den Einsatz 16 hindurch und endet im Bereich des distalen Endes 18 des Einsatzes 16 in einer Kanalmündung 36. In der Kanalmündung 36 befindet sich bei eingeschobener Endoskopoptik 30 deren Objektiv 39. Die Kanalmündung 36 befindet sich am proximalen Ende eines in dem distalen Ende 18 des Einsatzes 16 ausgebildeten Ausschnittes 37. Die Kanalmündung 36 und damit das Objektiv 39 der Endoskopoptik 30 befinden sich somit gegenüber dem äußersten distalen Ende leicht nach proximal versetzt.

Am proximalen Ende des Kanals 34 ist eine Optikkupplung 38 angeordnet, die eine Optikverriegelung 40 aufweist, um die Endoskopoptik 30 in dem Kanal 34 und damit an dem Einsatz 16 zu arretieren. Ferner ist im Bereich des Okulars 32 ein Stutzen 42 zum Anschluß eines hier nicht dargestellten Lichtleiters vorgesehen.

Im Bereich des distalen Endes 18 des Einsatzes 16 ist ein Schneidwerkzeug 48 angeordnet, das aus dem Einsatz 16 radial ausfahrbar und in diesen zurück einfahrbar ist. In Fig. 1 ist das Schneidwerkzeug 48 in seiner ausgefahrenen Position gezeigt, während Fig. 2 das Schneidwerkzeug 48 in seiner eingefahrenen Position zeigt. In der ausgefahrenen Position gemäß Fig. 1 ragt das Schneidwerkzeug 48 über den Umfang des Einsatzes 16 und über den Umfang des Tubus 12 hinaus. In der in Fig. 2 dargestellten eingefahrenen Position ist das Schneidwerkzeug 48 in dem Einsatz 16 vollständig versenkt. Ein hier nicht dargestellter zuschaltbarer Antrieb, bspw. ein Rotationsantrieb mit Exzenter, sorgt für eine hin- und hergehende Oszillationsbewegung des Schneidwerkzeuges 48 in der ausgefahrenen Position, und zwar in einer mit einem Doppelpfeil 49 angedeuteten Richtung.

Das Objektiv 39 der Endoskopoptik 30 ist bezüglich des Schneidwerkzeuges 48 hinter diesem und geringfügig seitlich versetzt angeordnet. Darüber hinaus ist das Objektiv 39 so ausgebildet, daß das Schneidwerkzeug 48 im Gesichtsfeld des Objektivs 39 liegt.

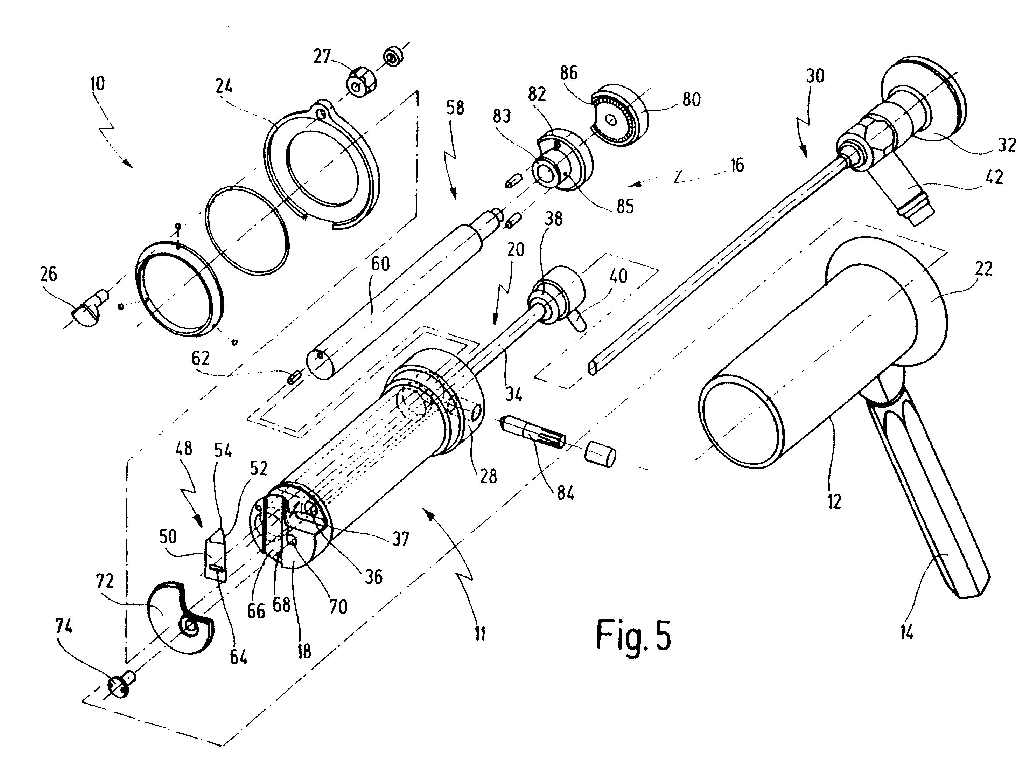

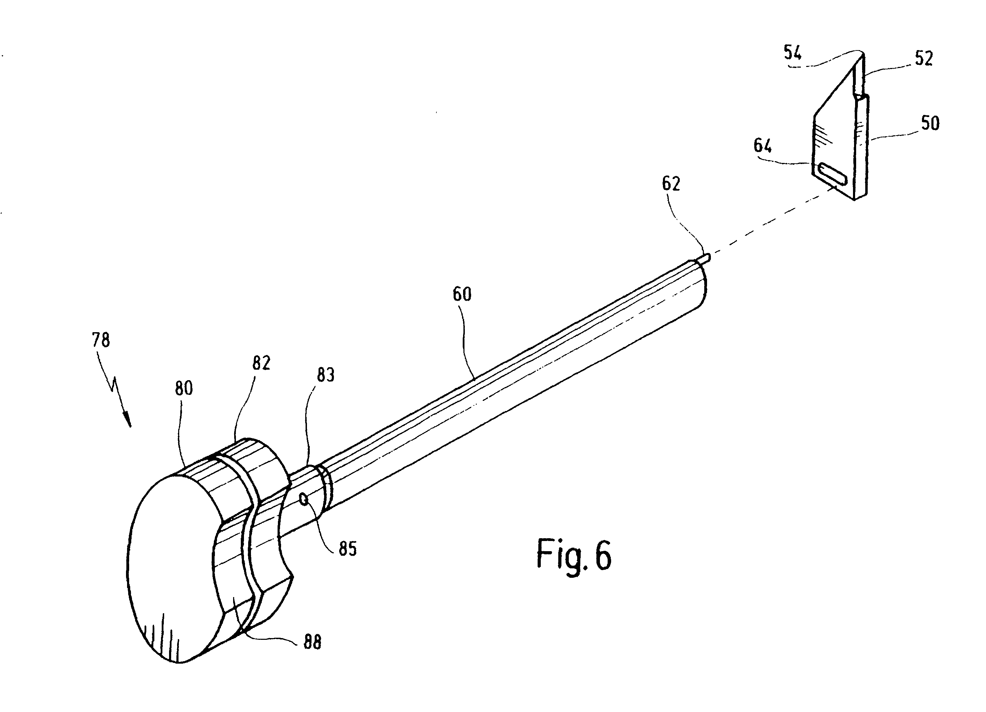

Das Schneidwerkzeug 48 besteht aus einer Klinge 50, die im wesentlichen in Form eines dünnen Metallplättchens ausgebildet ist (siehe Fig. 5 und 6). Die Klinge 50 weist eine Schneidkante 52 und eine Spitze 54 auf. Die Schneidkante 52 weist in ausgefahrenem Zustand der Klinge 50 in Umfangsrichtung des Einsatzes 16 (siehe Fig. 1).

Ferner ist zum Ein- und Ausfahren des Schneidwerkzeuges 48 ein mit dem allgemeinen Bezugszeichen 58 versehener Verstellmechanismus vorgesehen. Der Verstellmechanismus 58 weist einen Betätigungsstab 60 auf, der in den Einsatz 16 einschiebbar und aus diesem vollständig herausnehmbar ist. Der Betätigungsstab 60 ist in Fig. 5 und 6 im Detail dargestellt. An seinem distalen Ende weist der Betätigungsstab 60 einen axial vorstehenden und exzentrisch zur Längsachse angeordneten Stift 62 auf. Im vollkommen eingeschobenen Zustand des Betätigungsstabes 60 greift der Stift 62 in ein in der Klinge 50 ausgespartes Langloch 64 ein.

Die Klinge 50 ist in einer am distalen Ende 18 des Einsatzes 16 ausgesparten Ausnehmung 66 aufgenommen und darin radial verschiebbar. Die Ausnehmung 66 ist in axialer Richtung gerade so tief, wie die Klinge 50 dick ist. Die Ausnehmung 66 geht im wesentlichen radial durch das distale Ende 18 des Einsatzes 16 hindurch, ist somit von Umfang zu Umfang offen. Im Bereich des in Fig. 1 unteren Umfanges ist in der Ausnehmung 66 ein stiftförmiger Anschlag 68 für die Klinge 50 angeordnet, um die radiale Verschiebbarkeit der Klinge 50 an dem Anschlag 68 zu begrenzen.

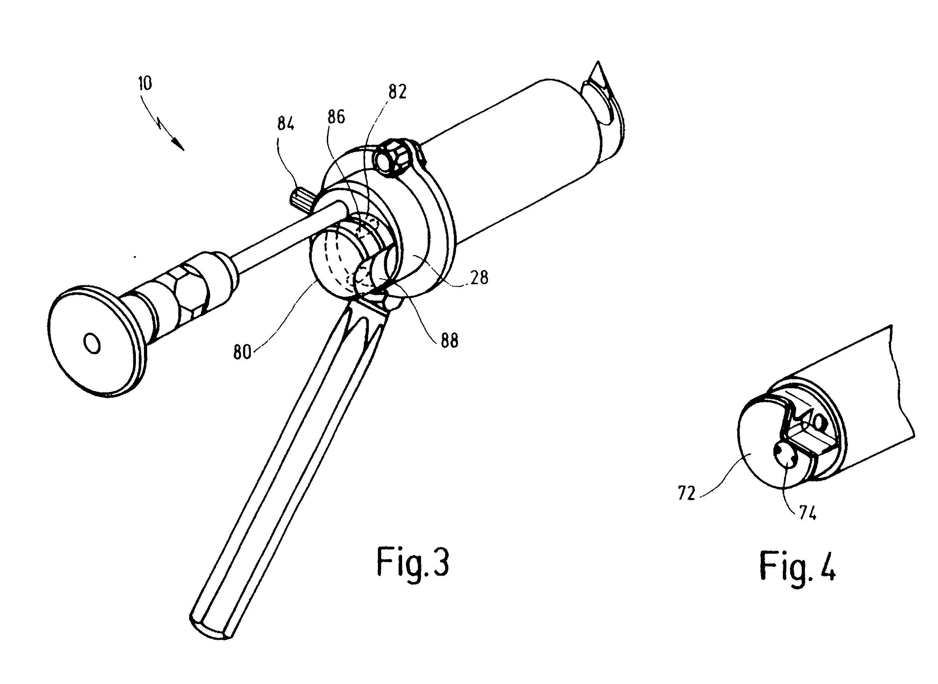

Ferner ist am distalen Ende 18 des Einsatzes 16 eine Gewindebohrung 70 vorgesehen, in die zum Abschließen des distalen Endes 18 des Einsatzes 16 ein Deckel 72 mit einer Schraube 74 befestigt werden kann, wie in Fig. 4 dargestellt ist. Bei verschlossenem Deckel 72 bildet die Ausnehmung 66 zusammen mit dem Deckel 72 einen Schlitz, in dem die Klinge 50 nur radial beweglich ist.

An seinem proximalen Ende weist der Betätigungsstab 60 zwei relativ zueinander verdrehbare Räder 80 und 82 auf, wobei das Rad 80 drehfest mit dem Bestätigungsstab 60 verbunden ist, während das Rad 82 um die Längsachse des Betätigungsstabes 60 drehbar ist. Dazu weist das Rad 82 einen axialen Fortsatz in Form einer Hülse 83 auf, die auf den Betätigungsstab 60 drehbar befestigt ist (vgl. Fig. 5).

In dem großen gerändelten Rad 28 des Einsatzes 16 ist eine Arretierschraube 84 angeordnet, mit der das Rad 82 des Betätigungsstabes 60 an dem Einsatz 16 arretiert werden kann. Dazu ist in der Hülse 83 des Rades 82 eine Bohrung vorgesehen, in die ein Ende der Arretierschraube 84 eingreift. Bei eingedrehter Arretierschraube 84 ist der Betätigungsstab 60 axial an dem Einsatz 16 fixiert, und zusätzlich ist das Rad 82 an dem Einsatz 16 drehfest fixiert.

Zwischen dem Rad 80 und dem Rad 82 sind mehrere Kugelrasten 86 angeordnet, die aus zwei diametral zueinander in dem Rad 82 federnd gelagerten Kugeln und einer Vielzahl von entsprechenden Kugelkalotten in dem Rad 80 gebildet sind (siehe Fig. 5). Beim Verdrehen des Rades 80 bei gleichzeitig fixiertem Rad 82 kann der Betätigungsstab 60 zum Ein- und Ausfahren der Klinge 50 gedreht werden, wobei aufgrund der Kugelrasten 86 das Rad 80 und damit der Betätigungsstab 60 in einer Vielzahl von Drehstellungen verrastbar ist. Unterschiedliche Drehstellungen des Rades 80 bewirken unterschiedliche Ausfahrweiten der Klinge 50, wie noch hiernach näher erläutert wird.

Ferner ist an dem Rad 80 eine Anzeigevorrichtung in Form einer Skala vorgesehen, mit der die Drehstellung und damit die Ausfahrweite der Klinge 50 genau abgelesen werden kann. Zusätzlich oder alternativ ist an der Klinge 50 eine Skala vorhanden, die die jeweilige Ausfahrweite der Klinge 50 angibt.

In den Rädern 80 und 82 ist ferner jeweils ein identischer kreisförmiger Ausschnitt 88 ausgebildet.

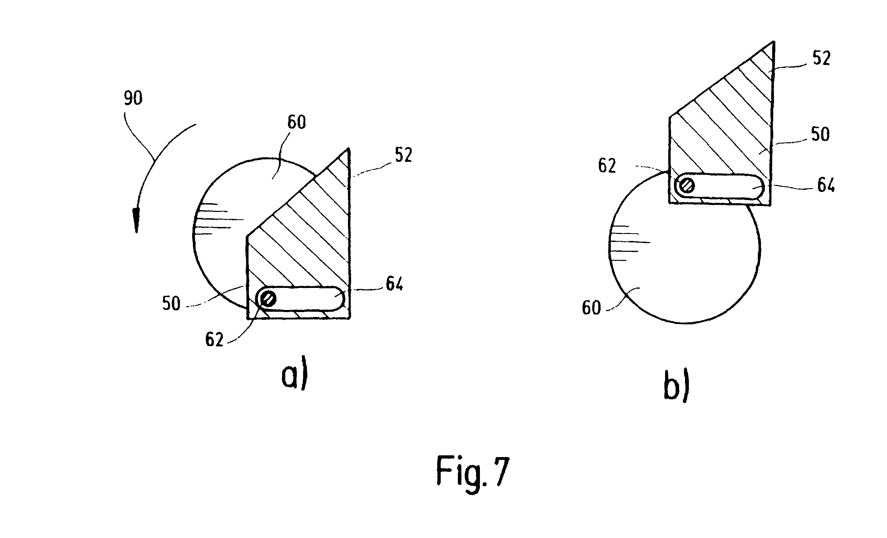

Mit Bezug auf Fig. 7a) und b) wird nun die Funktionsweise des Verstellmechanismus 58 beschrieben.

Fig. 7a) zeigt die Klinge 50 in der vollständig eingefahrenen Position gemäß Fig. 2 und 4. Im komplett montierten und betriebsfertigen Zustand des Proktoskops 10 greift, wie bereits erwähnt, der Stift 62 des Betätigungsstabes 60 in das Langloch 64 der Klinge 50 ein. Die Klinge 50 liegt dann auf dem Anschlag 68 in der Ausnehmung 66 auf (vgl. Fig. 2). Wird nun der Betätigungsstab 60 über das Rad 80 in Richtung eines Pfeiles 90 gedreht, fährt dabei die Klinge 50 aus der Ausnehmung 66 über den Umfang des Einsatzes 16 bzw. des Tubus 12 hinaus aus, wobei in Fig. 7b) die Klinge 50 in einer Position maximaler Ausfahrweite dargestellt ist. Die Klinge 50 fährt aus der Ausnehmung 66 in radialer Richtung aus. Zwischen der in Fig. 7a) dargestellten vollkommen eingefahrenen Position und der in Fig. 7b) dargestellten maximalen ausgefahrenen Position kann die Klinge 50 jede Zwischenposition mit unterschiedlicher Ausfahrweite einnehmen, wobei die Klinge 50 aufgrund der Kugelrasten 86 zwischen den Rädern 80 und 82 in einer Vielzahl von Positionen unterschiedlicher Ausfahrweite verrastbar und damit arretierbar ist. Um die Klinge 50 von der eingefahrenen in die maximal ausgefahrene Position zu bringen, ist eine Drehung des Rades 80 von etwa 180° erforderlich. Zum Einfahren der Klinge 50 wird das Rad 80 in umgekehrter Richtung zurückgedreht.

Im folgenden wird die Handhabung des Proktoskops 10 bei einem operativen Verfahren beschrieben.

Vor der Anwendung liegt der Einsatz 16 mit eingesetzter Endoskopoptik 30, eingesetztem Verstellmechanismus 58 und eingesetzter Klinge 50 getrennt von dem Tubus 12 vor. In den Tubus 12 wird ein hier nicht näher dargestellter Obturator mit einem konischen distalen Ende eingeschoben, und diese Anordnung aus Tubus 12 und Obturator wird in den Analkanal des Patienten eingeführt. Der Obturator wird sodann aus dem Tubus herausgezogen, und anstelle des Obturators wird der Einsatz 16 eingeführt. Beim Einsetzen des Einsatzes 16 ist das Schneidwerkzeug 48, hier die Klinge 50, in der vollständig eingefahrenen Stellung, in der die Spitze 54 der Klinge 50 nicht über den Umfang des Einsatzes hinausragt. Der Einsatz 16 wird vollständig in den Tubus 12 eingeschoben. Sodann wird der Einsatz 16 mit dem Befestigungsmittel 25 an dem Tubus 12 befestigt, indem das Klemmstück 26 über das konische proximale Ende des Tubus gebracht wird. Durch Drehen an dem großen gerändelten Rad 28 kann das Operationsfeld durch das Okular 32 hindurch gesucht und eingestellt werden. In der gewünschten Position wird das Proktoskop 10 mit der hier nicht dargestellten Haltevorrichtung über den Griff 14 fixiert. Durch Drehen am Rad 80 wird die Klinge 50 ausgefahren und der Schnitt kann durch Drehen des Einsatzes 16 an dem großen gerändelten Rad 28 kreisförmig parallel zum Rand des Proktoskops 10 vorgenommen werden. Sobald der Schnitt vorgenommen worden ist, wird die Klinge 50 durch Drehen des Rades 80 in umgekehrter Richtung wieder in den Einsatz 16 eingefahren, bis die Klinge 50 vollständig versenkt ist. Danach kann der Einsatz 16 aus dem Tubus 12 wieder herausgezogen werden.

Während dieser Operation wird über einen nicht dargestellten Spülkanal, der durch den Schaft 11 bis zu dem Schneidwerkzeug 48 reicht und dort einen Spülaustritt aufweist, gespült. Dadurch kann die Sicht bei der Operation verbessert werden, um Blut und Gewebeteile von dem Objektiv 39 zu beseitigen.

Wahlweise kann über einen Insufflationskanal durch den Schaft 11, insbesondere bei einer Operation in Körperkanälen mit kleinerem Durchmesser, wie bspw. der Speiseröhre, ein gasförmiges Medium in den Körperkanal insuffliert werden, um eine Kanalerweiterung und damit eine Sichtverbesserung zu erreichen.

Schließlich wird nun das Zerlegen des Einsatzes 16 und das Aufwechseln der Klinge 50 beschrieben. Die Endoskopoptik 30 wird von dem Einsatz 16 entfernt, indem die Optikverriegelung 40 gelöst, und die Endoskopoptik 30 aus dem Kanal 34 durch die Optikkupplung 38 herausgezogen wird.

Als nächstes wird die Arretierschraube 84 gelöst. Der Betätigungsstab 60 wird dann etwa um die Länge der Hülse 83 proximal zurückgezogen, so daß der Stift 62 mit dem Langloch 64 außer Eingriff kommt. Durch Drehen des Einsatzes 16 fällt dann die Klinge 50 aus dem Schlitz, der durch die Ausnehmung 66 und den Deckel 72 gebildet wird, heraus. Zum Herausnehmen der Klinge 50 ist es somit nicht erforderlich, den Deckel 72 zu entfernen. Der Betätigungsstab 60 kann vollständig aus dem Einsatz 16 herausgezogen werden, wenn die kreisförmigen Ausschnitte 88 der beiden Räder 80 und 82 in Deckung gebracht werden. Dann nämlich lassen sich die Räder 80 und 82 bei herausgenommener Endoskopoptik 30 an der Optikkupplung 38 vorbei proximal aus dem Einsatz 16 herausziehen.

Zum Einsetzen der Klinge 50 wird zunächst der Betätigungsstab 60 in den Einsatz 16 eingeführt, und zwar bis zu der Hülse 83.

Danach wird die Klinge 50 in die Ausnehmung 66 fallengelassen, die sich dann auf dem Anschlag 68 abstützt. Der Betätigungsstab 60 kann nun vollständig in den Einsatz 16 eingeschoben werden, indem der Betätigungsstab 60 solange verdreht wird, bis der Stift 62 auf gleicher Höhe wie das Langloch 64 positioniert ist. Dann läßt sich der Betätigungsstab 60 vollkommen in den Einsatz 16 einschieben, wobei der Stift 62 in das Langloch 64 eingesteckt wird, und die Arretierschraube 84 wird zum Arretieren des Rades 82 in die Bohrung 85 eingeschraubt. Der Einsatz 16 ist sodann betriebsbereit.

## Patentansprüche

1. Endoskopisches Instrument, mit einem Schaft (11) zum Einführen in kanalartige Körpergänge, wie Rektum, Trachea, Ösophagus, sowie mit einer Endoskopoptik (30), **dadurch gekennzeichnet, daß** in dem Schaft (11) ein Schneidwerkzeug (48) zum Einbringen eines Schnittes in umfänglicher Richtung des Körperganges angeordnet ist, das über einen Verstellmechanismus (58) aus dem Schaft (11) über den Umfang des Schafts (11) hinaus ausfahrbar und wieder einfahrbar ist.

2. Instrument nach Anspruch 1, **dadurch gekennzeichnet, daß** das Schneidwerkzeug (48) in der maximal ausgefahrenen und in der eingefahrenen Position arretierbar ist.

3. Instrument nach Anspruch 1 oder 2, **dadurch gekennzeichnet, daß** das Schneidwerkzeug (48) in einer Vielzahl von Positionen unterschiedlicher Ausfahrweiten zwischen der eingefahrenen und der maximal ausgefahrenen Position arretierbar ist.

4. Instrument nach einem der Ansprüche 1 bis 3, **dadurch gekennzeichnet, daß** das Schneidwerkzeug (48) radial ausfahrbar ist.

5. Instrument nach einem der Ansprüche 1 bis 4, **dadurch gekennzeichnet, daß** der Verstellmechanismus (58) vom proximalen Ende des Instruments (10) aus betätigbar ist.

6. Instrument nach einem der Ansprüche 1 bis 5, **dadurch gekennzeichnet, daß** der Verstellmechanismus (58) zum Arretieren des Schneidwerkzeuges (48) verrastbar ist.

7. Instrument nach einem der Ansprüche 1 bis 6, **dadurch gekennzeichnet, daß** der Verstellmechanismus (58) einen proximal betätigbaren Betätigungsstab (60) aufweist, dessen distales Ende mit dem Schneidwerkzeug (48) zusammenwirkt.

8. Instrument nach Anspruch 7, **dadurch gekennzeichnet, daß** der Betätigungsstab (60) drehbar ist und das Schneidwerkzeug (48) durch Drehen des Betätigungsstabes (60) aus- und einfahrbar ist.

9. Instrument nach Anspruch 8, **dadurch gekennzeichnet, daß** das proximale Ende des Betätigungsstabes (60) zwei relativ zueinander verdrehbare Räder (80, 82) aufweist, wobei das eine Rad (80) drehfest mit dem Betätigungsstab (60) verbunden ist und das andere Rad (82) drehfest an dem Schaft (11) befestigbar ist.

10. Instrument nach Anspruch 8 oder 9, **dadurch gekennzeichnet, daß** zwischen den Rädern (80, 82) eine oder mehrere Kugelrasten (86) angeordnet sind.

11. Instrument nach Anspruch 7, **dadurch gekennzeichnet, daß** der Betätigungsstab (60) axial verschiebbar ist und das Schneidwerkzeug (48) durch Verschieben des Betätigungsstabes (60) ein- und ausfahrbar ist.

12. Instrument nach einem der Ansprüche 7 bis 9, **dadurch gekennzeichnet, daß** der Betätigungsstab (60) mit dem Schneidwerkzeug (48) durch eine axial lösbare Steckverbindung verbunden ist.

13. Instrument nach einem der Ansprüche 1 bis 12, **dadurch gekennzeichnet, daß** ein Objektiv (39) der Endoskopoptik (30) im Bereich des Schneidwerkzeuges (48) derart angeordnet ist, daß das Schneidwerkzeug (48) durch die Endoskopoptik (30) beobachtbar ist.

14. Instrument nach einem der Ansprüche 1 bis 13, **dadurch gekennzeichnet, daß** das Schneidwerkzeug (48) in der ausgefahrenen Position über einen Antrieb in Oszillation versetzbar ist.

15. Instrument nach einem der Ansprüche 1 bis 14, **dadurch gekennzeichnet, daß** eine Anzeigevorrichtung für die Ausfahrweite des Schneidwerkzeuges (48) vorgesehen ist.

16. Instrument nach Anspruch 15, **dadurch gekennzeichnet, daß** die Anzeigevorrichtung am Schneidwerkzeug (48) vorgesehen ist.

17. Instrument nach Anspruch 15 oder 16, **dadurch gekennzeichnet, daß** die Anzeigevorrichtung am Verstellmechanismus (58) vorgesehen ist.

18. Instrument nach einem der Ansprüche 1 bis 17, **dadurch gekennzeichnet, daß** das Schneidwerkzeug (48) eine Klinge (50) ist, deren Schneidkante (52) in Umfangsrichtung des Schafts (11) gerichtet ist.

19. Instrument nach Anspruch 18, **dadurch gekennzeichnet, daß** sich die Klinge (50) im Bereich der Schneidkante (52) zu einer Spitze (54) verjüngt.

20. Instrument nach Anspruch 18 oder 19, **dadurch gekennzeichnet, daß** die Klinge (50) ein Langloch (64) aufweist, in das ein am distalen Ende des Betätigungsstabes (60) exzentrisch zu dessen Längsachse angeordneter Stift (62) eingreift.

21. Instrument nach einem der Ansprüche 18 bis 20, **dadurch gekennzeichnet, daß** die Klinge (50) in einer zum Umfang des Schafts (11) hin offenen, im wesentlichen radial verlaufenden Ausnehmung (66) des Schafts (11) aufgenommen ist.

22. Instrument nach einem der Ansprüche 1 bis 21, **dadurch gekennzeichnet, daß** der Schaft (11) als Einsatz (16) ausgebildet ist, der in einen Tubus (12) einschiebbar ist.

23. Instrument nach Anspruch 22, **dadurch gekennzeichnet, daß** der Einsatz (16) in dem Tubus (22) drehbar befestigbar ist.

24. Instrument nach einem der Ansprüche 1 bis 17, **dadurch gekennzeichnet, daß** das Schneidwerkzeug (48) ein Schneidedraht ist.

25. Instrument nach einem der Ansprüche 1 bis 24, **dadurch gekennzeichnet, daß** der Schaft (11) flexibel ist.

## Claims

1. An endoscopic instrument, comprising a shaft (11) for introduction into duct-like body passages such as the rectum, trachea, or esophagus, and comprising an endoscopic optical system (30), **characterized in that** there is arranged in the shaft (11) a cutting tool (48) for making an incision in the circumferential direction of the body passage, which cutting tool, by way of a displacement mechanism (58), can be extended out of the shaft (11) beyond the periphery of the shaft (11) and retracted back thereinto.

2. The instrument as defined in claim 1, **characterized in that** the cutting tool (48) is lockable in the maximally extended position and in the retracted position.

3. The instrument of claim 1 or 2, **characterized in that** the cutting tool (48) is lockable in a plurality of positions of different extension spans between the retracted and maximally extended positions.

4. The instrument of anyone of claims 1 through 3, **characterized in that** the cutting tool (48) can be extended radially.

5. The instrument of anyone of claims 1 through 4, **characterized in that** the displacement mechanism (58) is actuable from the proximal end of the instrument (10).

6. The instrument of anyone of claims 1 through 5, **characterized in that** the displacement mechanism (58) can be snaplocked for locking the cutting tool (48).

7. The instrument of anyone of claims 1 through 6, **characterized in that** the displacement mechanism (58) has a proximally actuable actuation rod (60) whose distal end coacts with the cutting tool (48).

8. The instrument of claim 7, **characterized in that** the actuation rod (60) is rotatable, and the cutting tool (48) can be extended and retracted by rotating the actuation rod (60).

9. The instrument of claim 8, **characterized in that** the proximal end of the actuation rod (60) has two wheels (80, 82) that are rotatable relative to one another, the one wheel (80) being joined to the actuation rod (60) in a rotationally fixed manner and the other wheel (82) being attachable on the shaft (11) in a rotationally fixed manner.

10. The instrument of claim 8 or 9, **characterized in that** one or more ball catches (86) are arranged between the wheels (80, 82).

11. The instrument of claim 7, **characterized in that** the actuation rod (60) is axially displaceable and the cutting tool (48) can be extended and retracted by displacing the actuation rod (60).

12. The instrument of anyone of claims 7 through 9, **characterized in that** the actuation rod (60) is joined to the cutting tool (48) by way of an axially detachable plug-in connection.

13. The instrument of anyone of claims 1 through 12, **characterized in that** an objective lens (39) of the endoscopic optical system (30) is arranged in the region of the cutting tool (48) in such a way that the cutting tool (48) is observable through the endoscopic optical system (30).

14. The instrument of anyone of claims 1 through 13, **characterized in that**, in the extended position, the cutting tool (48) can be caused to oscillate by way of a drive system.

15. The instrument of anyone of claims 1 through 14, **characterized in that** an indicating apparatus for the extension span of the cutting tool (48) is provided.

16. The instrument of claim 15, **characterized in that** the indicating apparatus is provided on the cutting tool (48).

17. The instrument of claim 15 or 16, **characterized in that** the indicating apparatus is provided on the displacement mechanism (58).

18. The instrument of anyone of claims 1 through 17, **characterized in that** the cutting tool (48) is a blade (50) whose cutting edge (52) is oriented in the circumferential direction of the shaft (11).

19. The instrument of claim 18, **characterized in that** the blade (50) tapers to a tip (54) in the region of the cutting edge (52).

20. The instrument of claim 18 or 19, **characterized in that** the blade (50) has an oblong hole (64) into which engages a pin (62) arranged at the distal end of the actuation rod (60) eccentrically with respect to the longitudinal axis thereof.

21. The instrument of anyone of claims 18 through 20, **characterized in that** the blade (50) is received in a recess (66) of the shaft (11) that is open toward the periphery of the shaft (11) and extends substantially radially.

22. The instrument of anyone of claims 1 through 21, **characterized in that** the shaft (11) is configured as an insert (16) that can be slid into a tube (12).

23. The instrument of claim 22, **characterized in that** the insert (16) is attachable rotatably in the tube (22).

24. The instrument of anyone of claims 1 through 17, **characterized in that** the cutting tool (48) is a cutting wire.

25. The instrument of anyone of claims 1 through 24, **characterized in that** the shaft (11) is flexible.

## Revendications

1. Instrument endoscopique, avec une tige (11) pour une introduction dans des voies corporelles de type canal, comme le rectum, la trachée, l'oesophage, ainsi qu'avec une optique d'endoscope (30), **caractérisé en ce qu'**un outil de coupe (48) est placé dans la tige (11) pour réaliser une découpe dans la direction circonférentielle de la voie corporelle, qui peut être sorti de la tige (11), puis de nouveau rentré, sur la circonférence de la tige (11) par l'intermédiaire d'un dispositif de manoeuvre (58).

2. Instrument selon la revendication 1, **caractérisé en ce que** l'outil de coupe (48) peut être immobilisé dans la position sortie au maximum et dans la position rentrée.

3. Instrument selon la revendication 1 ou 2, **caractérisé en ce que** l'outil de coupe (48) peut être immobilisé dans une pluralité de positions correspondant à différentes distances de sortie entre la position rentrée et la position sortie au maximum.

4. Instrument selon l'une des revendications 1 à 3, **caractérisé en ce que** l'outil de coupe (48) peut être sorti radialement.

5. Instrument selon l'une des revendications 1 à 4, **caractérisé en ce que** le mécanisme de manoeuvre (58) peut être actionné depuis l'extrémité proximale de l'instrument (10).

6. Instrument selon l'une des revendications 1 à 5, **caractérisé en ce que** le mécanisme de manoeuvre (58) peut être enclenché pour immobiliser l'outil de coupe (48).

7. Instrument selon l'une des revendications 1 à 6, **caractérisé en ce que** le mécanisme de manoeuvre (58) présente une barre d'actionnement (60) actionnable du côté proximal, dont l'extrémité distale coopère avec l'outil de coupe (48).

8. Instrument selon la revendication 7, **caractérisé en ce que** la barre d'actionnement (60) est rotative et l'outil de coupe (48) peut être sorti et rentré par rotation de la barre d'actionnement (60).

9. Instrument selon la revendication 8, **caractérisé en ce que** l'extrémité proximale de la barre d'actionnement (60) présente deux roues (80, 82) pouvant tourner l'une par rapport à l'autre, la première roue (80) étant reliée de manière solidaire en rotation à la barre d'actionnement (60) et l'autre roue (82) pouvant se fixer de manière solidaire en rotation sur la tige (11).

10. Instrument selon la revendication 8 ou 9, **caractérisé en ce qu'**un ou plusieurs crans à billes (86) sont placés entre les roues (80, 82).

11. Instrument selon la revendication 7, **caractérisé en ce que** la barre d'actionnement (60) est mobile axialement et l'outil de coupe (48) peut être rentré et sorti par translation de la barre d'actionnement (60).

12. Instrument selon l'une des revendications 7 à 9, **caractérisé en ce que** la barre d'actionnement (60) est reliée à l'outil de coupe (48) par un connecteur désolidarisable axialement.

13. Instrument selon l'une des revendications 1 à 12, **caractérisé en ce qu'**un objectif (39) de l'optique d'endoscope (30) est placé au niveau de l'outil de coupe (48), de telle manière que l'outil de coupe (48) est observable à travers l'optique d'endoscope (30).

14. Instrument selon l'une des revendications 1 à 13, **caractérisé en ce que** l'outil de coupe (48) peut être mis en oscillation dans la position sortie par l'intermédiaire d'un entraînement.

15. Instrument selon l'une des revendications 1 à 14, **caractérisé en ce qu'**il est prévu un dispositif d'affichage de la distance de sortie de l'outil de coupe (48).

16. Instrument selon la revendication 15, **caractérisé en ce que** le dispositif d'affichage est prévu sur l'outil de coupe (48).

17. Instrument selon la revendication 15 ou 16, **caractérisé en ce que** le dispositif d'affichage est prévu sur le mécanisme de manoeuvre (58).

18. Instrument selon l'une des revendications 1 à 17, **caractérisé en ce que** l'outil de coupe (48) est une lame (50) dont le tranchant (52) est dirigé dans la direction circonférentielle de la tige (11).

19. Instrument selon la revendication 18, **caractérisé en ce que** la lame (50) est effilée en une pointe (54) au niveau du tranchant (52).

20. Instrument selon la revendication 18 ou 19, **caractérisé en ce que** la lame (50) présente un trou oblong (64) dans lequel s'engage un ergot (62) placé à l'extrémité distale de la barre d'actionnement (60) de manière excentrée par rapport à son axe longitudinal.

21. Instrument selon l'une des revendications 18 à 20, **caractérisé en ce que** la lame (50) est logée dans un évidement (66) de la tige (11) ouvert en direction de la circonférence de la tige (11) et d'extension essentiellement radiale.

22. Instrument selon l'une des revendications 1 à 21, **caractérisé en ce que** la tige (11) est conformée en insert (16) qui peut être enfoncé dans un tube (12).

23. Instrument selon la revendication 22, **caractérisé en ce que** l'insert (16) peut être fixé de manière rotative dans le tube (22).

24. Instrument selon l'une des revendications 1 à 17, **caractérisé en ce que** l'outil de coupe (48) est un fil coupant.

25. Instrument selon l'une des revendications 1 à 24, **caractérisé en ce que** la tige (11) est flexible.
